# DEMANDE DE BREVET EUROPEEN

(11) **EP 0 205 365 A1**
(43) Date de publication de la demande: **17.12.1986**
(21) Numéro de dépôt: 86401012.9
(22) Date de dépôt: 12.05.1986
(51) Int. Cl.: C07C 69/675, C07C 67/38

(54) **Procédé d'hydrocarbo-alcoxylation d'alcanoates de vinyle**

(30) Priorité: 11.06.1985 FR 8508939
(71) Demandeur: INSTITUT FRANCAIS DU PETROLE, F-92502 Rueil-Malmaison (FR)
(72) Inventeur: Chauvin, Yves, F-78230 le Pecq (FR); Commereuc, Dominique, F-92190 Meudon (FR); Hugues, François, F-92000 Nanterre (FR)

(57) **Abrégé**

L'invention concerne un procédé de préparation d'acyloxy-3-alcanoates d'alkyle par réaction entre un alcanoate de vinyle, un gaz contenant du monoxyde de carbone et un alcool en présence d'un catalyseur renfermant'du cobalt ou un composé de cobalt.

Le procédé consiste à faire réagir en phase liquide un alcanoate de vinyle avec du monoxyde de carbone en présence d'un alcool, d'un catalyseur au cobalt et de préférence d'un cocatalyseur choisi parmi les bases azotées tertiaires, ou les N-oxydes des bases azotées tertiaires.

Le catalyseur au cobalt est de préférence un composé de cobalt pouvant former in-situ un complexe carbonyle du cobalt.

Le gaz contenant du monoxyde de carbone contient également de préférence de 0,1 à 20% d'hydrogène.

L'acétoxy-3-propionate de méthyle est obtenu à partir de l'acétate de vinyle et du méthanol. Les alcyloxy-3-alkanoates d'alkyle sont aisément transformables, par simple chauffage, en esters d'acide acrylique.

## Description

L'objet de la présente invention est un procédé de préparation d'alcyloxy-3-alcanoates d'alkyle par hydrocarbo-alcoxylation d'alcanoates de vinyle en présence d'un catalyseur à base d'un métal du groupe VIII de la classification périodique des éléments.

Au sens de la présente invention, on appelle procédé d'hydrocarbo-alcoxylation d'alcanoates de vinyle, un procédé qui consiste à faire réagir un alcanoate de vinyle avec un gaz contenant du monoxyde de carbone, en présence d'un alcool.

Ce procédé permet en particulier d'obtenir à partir de l'acétate de vinyle des esters de l'acide acétoxy-3 propanoïque Les esters des acides acétoxylés obtenus peuvent être facilement craqués en esters d'acides insaturés, et on peut ainsi en particulier obtenir des esters de l'acide acrylique par craquage des esters de l'acide acétoxy-3-propanoïque.

On peut également transformer facilement les esters d'acides acétoxylés obtenus en esters d'acides hydroxylés correspondants. Alors que la carbo- nylation réductrice ou hydroformylation des alcanoates de vinyle et en particulier de l'acétate de vinyle a été largement étudiée (J. FALBE,Ne_w Syn- theses with Carbon Monoxide, Spinger Verlag-1980, p.122 et suivantes); l'hydrocarboxylation des alcanoates de vinyle, c'est-à-dire la réaction du monoxyde de carbone sur un alcanoate de vinyle en présence d'eau, et l'hydrocarbo-alcoxylation des alcanoates de vinyle n'ont pratiquement pas été étudiées.

Le brevet français FR-B-2 405 919 correspondant au brevet US-A-4 377 708 décrit un procédé d'hydrocarboxylation de l'acétate de vinyle en présence d'eau et d'un catalyseur au palladium stabilisé par une organophosphine tertiaire. Ce procédé permet d'obtenir de l'acide acétoxy-2-propanoïque qui peut ensuite être transformé par hydrolyse en acide lactique.

L'objet de la présente invention est-un procédé d'hydrocarboalcoxylation d'alcanoates de vinyle qui consiste à faire réagir, en phase liquide, dans des conditions substantiellement anhydre, un alcanoate. de vinyle avec un gaz contenant du monoxyde de carbone et avec un alcool en présence d'un catalyseur renfermant au moins du cobalt ou composé de cobalt.

On a en effet découvert que la réaction du monoxyde de carbone sur les alcanoates de vinyle en présence d'un alcool en proportion convenable permet d'obtenir des esters d'acides acyloxy-3-carboxyliques.

Le schéma de réaction s'écrit :

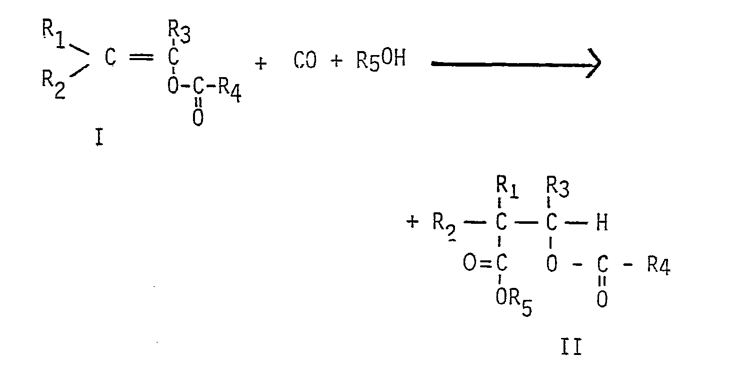

Dans les formules ci-dessus R,, R₂, R₃ et R, représentent indépendamment les uns des autres, un atome d'hydrogène ou un groupe hydrocarbyle ayant par exemple de 1 à 30 atomes de carbone. Parmi les groupes hydrocarbyles, on peut citer à titre d'exemple, les groupes alkyles ayant de 1 à 12 atomes de carbone, les groupes cycloalkyles ayant de 3 à 12 atomes de carbone, les groupes aryles ayant de 6 à 14 atomes de carbone et les groupes aralkyles ayant de 7 à 20 atomes de carbone. Les groupes R,, R2 et R3 peuvent éventuellement être porteurs de groupements fonctionnels tels que par exemple les groupes alcoxy, esters et tous groupes fonctionnels qui ne gênent pas la réaction d'hydrocarbo-alcoxylation.

Le groupe Rₛ représente un groupe hydrocarbyle ayant de 1 à 30 atomes de carbone. A titre d'exemple de groupe Rₛ, on peut citer les groupes alkyles de C, à C₁₂, les groupes cycloalkyles de C₃ à C,2, les groupes aryles de C₆ à C₁₄ et les groupes aralkyles de C7 à C₂₀.

Parmi les groupes R,, R₂, R₃ R4 et R₅ ci-dessus, on peut citer à titre d'exemple les groupes suivants : méthyle, éthyle, propyle, butyle, pentyle, cyclo- pentyle, cyclohexyle, phenyle, naphtyle, tolyle, xy- lyle, mésityle, indanyle, cuményle, benzyle, diphénylméthyle , phénétyle et trityle.

Les composés de départ de formule I préférés sont ceux dans lesquels le groupe R,, représente un atome d'hydrogène ou un groupe méthyle, les groupes R₂ et R₃ représentent un atome d'hydrogène et le groupe R₄ représente un atome d'hydrogène ou un groupe alkyle de C, à C₄ et d'une façon la plus préférée un groupe méthyle.

L'alcool de formule RₛOH est de préférence un alcool aliphatique saturé ayant de 1 à 4 atomes de carbone tel que, par exemple, le méthanol, l'éthanol, le propanol-1, le propanol-2, les butanols et en particulier le n-butanol-1, l'isobutanol, le méthyl-1-propanol-1 et l'alcool tertiobutylique.

On peut citer à titre d'exemple non limitatifs la réaction d'un gaz contenant du monoxyde de carbone sur les mélanges suivants : acétate de vinyle-méthanol, acétate de vinyle-éthanol, acétate de vinyle-propanol, acétate de vinyle-n-butanol,propionate de vinyle-méthanol, propionate de vinyle-éthanol,propionate de vinyle-propanol, propionate de vinyle-n-butanol, butyrate de vinyle-méthanol, butyrate de vinyle-éthanol, butyrate de vinyle-propanol et butyrate de vinyle-n-butanol.

Le présent procédé est particulièrement intéressant dans le cas de l'hydrocarbo-alcoxylation de l'acétate de vinyle en présence d'un alcool aliphatique ayant de 1 à 4 atomes de carbone.

Les esters d'acide acétoxy-3-propanoïques obtenus peuvent être aisément convertis par exemple par simple chauffage en esters de l'acide acrylique.

Le schéma de réaction s'écrit :

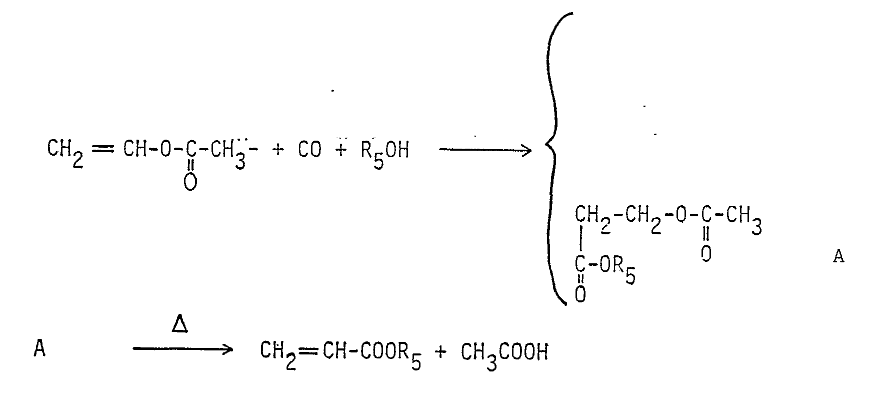

Dans les formules ci-dessus R₅ à la signification donnée supra. Le catalyseur nécessaire à la mise en oeuvre du procédé selon la présente invention, est un catalyseur renfermant au moins du cobalt ou un composé de cobalt. En plus du catalyseur on emploie de préférence au moins un cocatalyseur tel que défini ci-après.

Comme composé de cobalt, on considère tous les composés de cobalt et en particulier ceux susceptibles de réagir avec le monoxyde de carbone pour former in-situ au moins un complexe carbonyle du cobalt. Les composés de cobalt que l'on emploie sont par exemple le dicobalt octacarbonyle, le cobalt métallique à l'état très finement divisé, les sels de cobalt tels que par exemple les carboxylates et en particulier les acétates de cobalt, le nitrate de cobalt.

La concentration en cobalt exprimée par le rapport molaire cobalt/alcanoate de vinyle est de préférence d'environ d'environ 0,0001:1 à environ 0,1:1 et plus avantageusement d'environ 0,001:1 à. environ 0.05:1. En plus du composé de cobalt on emploie de préférence au moins un cocatalyseur choisi dans le groupe formé par les bases azotées tertiaires et leurs N-oxydes.

Parmi les bases azotées tertiaires ou leurs N-oxydes, il est avantageux d'employer des bases hétérocycliques azotées ou leurs N-oxydes, telles que par exemple la pyridine, les pyridines substituées par au moins un groupe alkyle, ledit groupe alkyle ayant par exemple de 1 à 10 atomes de carbone et de préférence de 1 à 4 atomes de carbone, la quinoléine, les quinoléïnes substi- tuéespar au moins un groupe alkyle tel que défini ci-dessus, l'isoquinoléine ou les isoquinoléines substituées par au moins un groupe alkyle tel que défini ci-dessus. Les composés hétérocycliques ci-dessus peuvent également être substitués par exemple par un groupe alcoxy ayant par exemple de 1 à 4 atomes de carbone. La quantité de base azotée tertiaire utilisée est de préférence telle que le rapport molaire base azotée tertiaire/cobalt est d'environ 0,5:1 à environ 50:1 et plus avantageusement d'environ 4:1 à environ 10:1.

La réaction d'hydrocarbo-alcoxylation est effectuée en présence d'une phase liquide dans des conditions substantiellement anhydres. La teneur en eau résiduelle dans le mélange de réaction ne doit pas dépasser environ 5% en poids et de préférence doit être inférieure à 1 % en poids.

On peut effectuer la réaction en l'absence de solvant ou en présence d'un solvant. Tout solvant présentant des propriétés d'inertie chimique et de stabilité dans les conditions de la-réacfion peut être employé. On peut,par exemple, utiliser un solvant organique choisi dans le groupe formé par les- hydrocarbures-saturés, les hydrocarbures aromati--- ques, les éthers, les composés carbonylés par exemple des cétones et les composés hétérocycliques oxygénés. Il est préférable de choisir un solvant qui peut être facilement séparé des produits obtenus.

Parmi les solvants préférés on peut citer à titre d'exemples non limitatifs le toluène, l'hexane, l'heptane, le diéthyléther, l'acétone, le dioxanne et le tetrahydrofurane.

La somme de la teneur en eau de l'alcool, de l'alconoate de vinyle, du monoxyde de carbone, et éventuellement du solvant que l'on introduit dans le réacteur sera de préférence inférieure à 0,5% en poids par rapport au poids total des produits utilisés dans la réaction.

Les conditions de pression, et de température bien que variables en fonction des substrats et du catalyseur sont généralement les suivantes :
-pression totale d'environ 1 à 30 MPa et avantageusement d'environ 5 à 30 MPa et de préférence d'environ 5 à 15 MPa,
-température d'environ 50 à 300°C et de préférence d'environ 80 à 180°C.

Le gaz contenant du monoxyde de carbone est le plus souvent un mélange monoxyde de carbone- hydrogène, bien que du monoxyde de carbone essentiellement pur puisse être utilisé également.

On emploie avantageusement un mélange monoxyde de carbonehydrogène contenant de 0,1 à environ 20% en volume d'hydrogène et 99,9 à environ 80% de monoxyde de carbone et de préférence d'environ 1 à environ 10% en volume d'hydrogène et 99 à 90% de monoxyde de carbone.

Le rapport molaire alcool (RₛOH)/alcanoate de vinyle est de préférence d'environ 1:1 à environ 50:1 et d'une façon avantageuse d'environ 1:1 à environ 10:1 et d'une manière la plus préférée d'environ 1:1 à environ 5:1. Il est possible d'opérer avec un large excès soit d'alcool,soit d'alcanoate de vinyle au cours de la réaction. On peut par exemple n'introduire qu'une faible proportion de l'alcool au début de la réaction de manière à avoir un rapport alcoollalcanoate de vinyle faible et introduire le reste de l'alcool progressivement ou par incréments au fur et à mesure du déroulement de la réaction.

De même, on peut n'introduire qu'une faible proportion d'alcanoate de vinyle au début de la réaction, le reste étant introduit progressivement ou par incréments au fur et à mesure du déroulement
-de la réaction. Dans ce cas, le rapport alcool/alcanoate de vinyle est constamment maintenu à une valeur élevée, mais variable.
- w- - Les exemples suivants illustrent l'invention sans en limiter la portée.

### Exemple 1

Dans un autoclave en acier inoxydable d'un volume utile de 300 cm3, équipé d'un dispositif de régulation de température par chauffage électrique et d'une agitation magnétique, on introduit 0,166 mole d'acétate de vinyle, 0,192 mole de n-butanol, 21,7 grammes de toluène, 20 millimoles de N-oxyde de méthyl-4-pyridine et 1,7 millimole de dicobaltoctacarbonyle.

On ferme l'autoclave et on le purge 3 fois par 1 MPa d'hydrogène.

On le pressurise ensuite à 10,4 MPa à 20°C avec un mélange d'hydrogène et de monoxyde de carbone (pression partielle d'hydrogène 1 MPa), puis on chauffe à 145°C.

Après 2 heures de réaction à 145°C, on refroidit l'autoclave jusqu'à la température ambiante. La baisse de pression relevée, ramenée aux conditions initiales, est de 1 MPa. On analyse le contenu liquide par chromatographie en phase vapeur - (CPV). La conversion de l'acétate de vinyle est de 83,1%. La sélectivité molaire , exprimée en pourcentage d'acétate de vinyle converti en acétoxy-3-propionate de butyle est de 31,2%.

### Exemple 2 (comparatif)

Dans le même appareillage que dans l'exemple 1, on introduit 0,475 mole de n-butanol, 4,3 millimoles de chlorure de palladium (PdCl₂) et 29,1 millimoles de triphenylphosphine. On purge 3 fois par 1 MPa de monoxyde de carbone contenant 1 % en volume d'hydrogène. On pressurise ensuite l'autoclave à 8 MPa à 20°C avec le mélange monoxyde de carbone-hydrogène à 1% d'hydrogène, puis on chauffe à 155°C.

On introduit alors l'acétate de vinyle à intervalles réguliers à raison de 2 millilitres toutes les 30 minutes environ, à partir d'un réservoir de liquide sous argon. maintenu à une pression supérieure à celle de l'autoclave. On a introduit au total environ 0,23 mole d'acétate de vinyle. Après 7 heures de réaction à 155°C, l'autoclave est alors refroidi jusqu'à la température ambiante. On analyse le contenu liquide par CPV. La conversion de l'acétate de vinyle est de 97% et la sélectivité molaire en acétoxy-3-propionate de butyle est de 0,4%.

### Exemples 2 à 5

Dans le même appareillage que-dans l'exem- pie 1, on met en réaction un alkanoate de vinyle et - un alcool avec un mélange monoxyde de carbone- hydrogène contenant 10% en volume d'hydrogène. Le catalyseur est à base de cobalt introduit dans le réacteur sous forme de dicobalt-octacarbonyle. Après 7 heures de réaction sous une pression de 10 MPa et une température de 150°C, l'autoclave est alors refroidi jusqu'à la température ambiante. On analyse le contenu liquide par CPV.

Le tableau 1 ci-après, regroupe les résultats obtenus exprimès par la conversion de l'alkanoate de vinyle et par la sélectivité molaire en pourcentage d'akanoate de vinyle converti en acyloxy-3-carboxylate d'alkyle.

### Exemple 6

Dans le même appareillage que dans l'exemple 1, on introduit 0,20 mole d'acétate de vinyle, 0,25 mole de butanol, 25 grammes de toluène, 20 millimoles de méthyl-4-pyridine et 3,8 millimoles d'acétate de cobalt Co (OAc)₂, 4H20.

On pressurise l'autoclave à 20 MPa à 20°C avec un mélange d'hydrogène et de monoxyde de carbone contenant 10% en volume d'hydrogène. On chauffe ensuite le mélange à 160°C pendant 1 heure ; après avoir refroidi l'autoclave à température ambiante on analyse le mélange réactionnel liquide.

La conversion de l'acétate de vinyle est de 98% et la sélectivité molaire en acetoxy-3-propio- noate de butyle est de 17%.

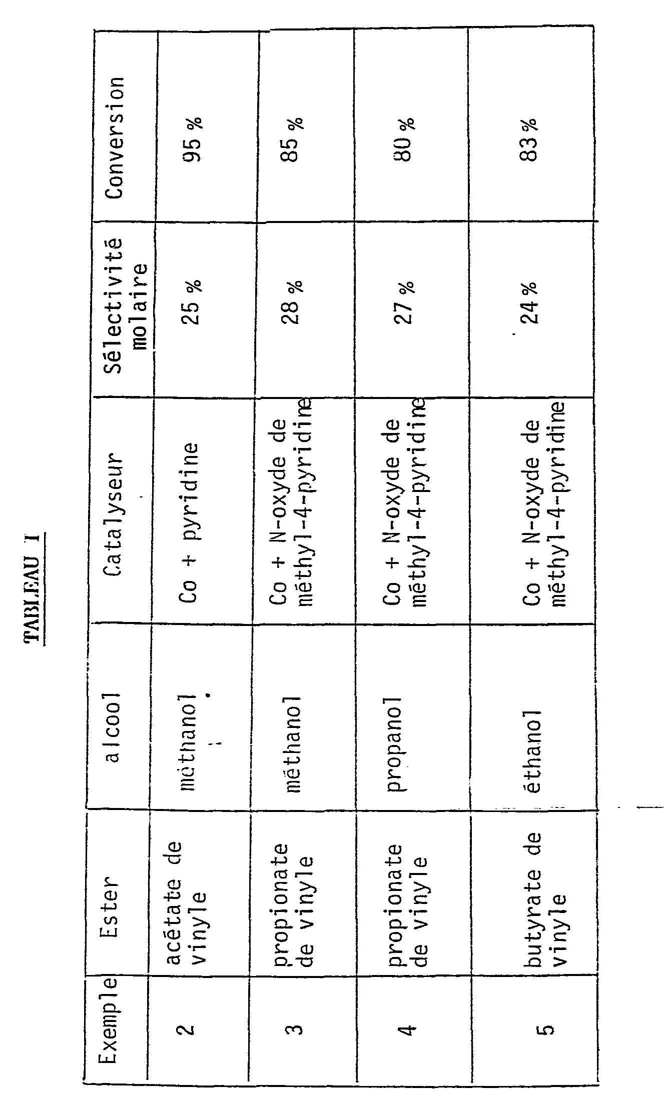

## Revendications

1. Procédé de préparation d'acyloxy-3-alcanoates d'alkyle caractérisé en ce qu'il consiste à faire réagir en phase liquide, dans des conditions substantiellement anhydres, un alcanoate de vinyle avec un gaz contenant du monoxyde de carbone et avec un alcool en présence d'un catalyseur renfermant du cobalt ou au moins un composé de cobalt et éventuellement au moins un cocatalyseur.

2. Procédé selon la revendication 1 dans lequel on emploie au moins un cocatalyseur choisi dans le groupe formé par les bases azotées tertiaires et les N-oxydes de bases azotées tertiaires.

3. Procédé selon la revendication 1 ou 2 dans lequel le catalyseur renferme au moins un composé de cobalt.

4. Procédé selon l'une des revendications 1 à 3 dans lequel le catalyseur renferme un composé de cobalt choisi parmi ceux pouvant former in-situ des complexes carbonyles du cobalt et un cocatalyseur choisi parmi les composés hétérocycliques azotés et les N-oxydes desdits composés.

5. Procédé selon la revendication 4 dans lequel le cocatalyseur est choisi dans le groupe formé par la pyridine, les alkylpyridines, la quinoleïne, les alkylquinole'lnes, l'isoquinoleÏne, les alkylisoquino- leÏnes et les N-oxydes desdits composés.

6. Procédé selon l'une des revendications 1 à 5 dans lequel le rapport molaire cobalt/alcanoate de vinyle est d'environ 0,0001 : 1 à environ 0.1 : 1 et le rapport molaire cocatalyseur/cobalt est d'environ 0,5 : 1 à environ 50 : 1.

7. Procédé selon l'une des revendications 1 à 6 dans lequel l'alcanoate de vinyle est l'acétate de vinyle.

8. Procédé selon l'une des revendications 1 à 7 dans lequel l'alcool est choisi dans le groupe formé par le méthanol, l'éthanol, les propanols et les butanols.

9. Procédé selon l'une des revendications 1 à 8 dans lequel on emploie un solvant choisi dans le groupe formé par les hydrocarbures aliphatiques, les hydrocarbures aromatiques, les éthers et les composés hétérocycliques oxygénés.

10. Procédé selon l'une des revendications 1 à 9 dans lequel le gaz employé est un mélange de monoxyde de carbone et d'hydrogène contenant en volume de 0,1 % à environ 20% d'hydrogène et de 99,9 à environ 80% de monoxyde de carbone.

11. Procédé selon l'une des revendications 1 à 10 dans lequel le rapport molaire alcool/alcanoate de vinyle est d'environ 1 : 1 à environ 50 : 1, la température de réaction est d'environ 50 à environ 300°C, la pression totale est d'environ 1 MPa à environ 30 MPa.
